# EUROPEAN PATENT SPECIFICATION

(11) **EP 2 948 772 B1**
(45) Date of publication and mention of the grant of the patent: **10.10.2018**
(21) Application number: 13872642.7
(22) Date of filing: 22.01.2013
(51) Int. Cl.: G01N 33/543, G01N 21/01, G01N 21/75, G02B 6/00, G01N 21/77, G01N 21/64

(54) **A HYDROGEL, FOR USE IN A BIOSENSOR WITH OPTICS BASED DETECTION OF ANALYTES**
HYDROGEL ZUR VERWENDUNG IN EINEM BIOSENSOR MIT OPTIKBASIERTEM NACHWEIS VON ANALYTEN
HYDROGEL DESTINÉ À ÊTRE UTILISÉ DANS UN BIOCAPTEUR DOTÉ D'UNE DÉTECTION DE TYPE OPTIQUE D'ANALYTES

(43) Date of publication of application: 02.12.2015
(73) Proprietor: Nitto Denko Corporation, Ibaraki-shi, Osaka 567-8680 (JP)
(72) Inventor: XIE, Hong, Singapore 138623 (SG); HAN, Jamie, Singapore 138623 (SG); KIBROM, Asmorom, 1190 Vienna (AT); KNOLL, Wolfgang, 1190 Vienna (AT); DOSTÁLEK, Jakub, 1190 Vienna (AT)
(74) Representative: Talbot-Ponsonby, Daniel Frederick
(86) International application number: PCT/SG2013/000033
(87) International publication number: WO 2014/116179

(56) References cited:
- WO-A1-01/55704
- WO-A2-2005/003821
- HUANG C J ET AL: "Long range surface plasmon and hydrogel optical waveguide field-enhanced fluorescence biosensor with 3D hydrogel binding matrix: On the role of diffusion mass transfer", BIOSENSORS AND BIOELECTRONICS, ELSEVIER BV, NL, vol. 26, no. 4, 15 December 2010 (2010-12-15), pages 1425-1431, XP027546859, ISSN: 0956-5663 [retrieved on 2010-08-19]
- TOMA, K. ET AL.: 'Bloch surface wave-enhanced fluorescence biosensor'.' BIOSENSORS AND BIOELECTRONICS vol. 43, 2013, pages 108 - 114, XP055264031
- RUBINA, A. Y. ET AL.: 'Quantitative immunoassay of biotoxins on hydrogel-based protein microchips'.' ANALYTICAL BIOCHEMISTRY vol. 340, 2005, pages 317 - 329, XP004853403
- NAGL, S. ET AL.: 'Microarray analysis of protein-protein interactions based on FRET using subnanosecond-resolved fluorescence lifetime imaging'.' BIOSENSORS AND BIOELECTRONICS vol. 24, 2008, pages 397 - 402, XP024100172
- XIE, M. ET AL.: 'Lectin-modified trifunctional nanobiosensors for mapping cell surface glycoconjugates'.' BIOSENSORS AND BIOELECTRONICS vol. 24, 2009, pages 1311 - 1317, XP025868523

## Description

### Field of the invention

The present invention relates to a hydrogel. The present invention also refers to a detection device comprising the hydrogel for measuring analyte levels in a sample fluid, and a method of measuring analyte levels.

### Background of the invention

Traditional assays for detection of molecular analytes such as enzyme-linked immunoassays (ELISA) involve multi-step sample processing prior to the detection such as addition, incubation and washing of detection agents. Accordingly, such assays become difficult to integrate into point-of-care (POC) systems.

While simple detection systems of molecular analytes such as strip-based technologies have been developed, it is not possible to quantify the amount of molecular analytes present in a sample. Further, such strip-based technology cannot be applied for multi-analyte detection in a straightforward manner.

WO 2011/146486 describes a microarray detection device which comprises a polymer layer with a capture region comprising capture agent and a labile region comprising detection agent and an excipient, wherein the capture region and labile region are spatially separated. Such a separation increases the cost of fabrication the sensing area. Further, a brush polymer layer is attached to a solid support through a functional group at one end of the polymer layer and hence, the mechanical stability of the polymer layer is weak.

There is therefore a need for an improved device which allows fast and quantitative detection of analytes with minimum or no sample pre-processing steps.

WO 2005/003821 discloses a phase change sensor. "Long range surface plasmon and hydrogel optical waveguide field-enhanced fluorescence biosensor with 3D hydrogel binding matrix: On the role of diffusion mass transfer", BIOSENSORS AND BIOELECTRONICS, ELSEVIER BV, NL, vol. 26, no. 4, 15 December 2010 by HUANG C J ET AL describes an implementation of a biosensor.

### Summary of the invention

The present invention seeks to address at least one of the problems in the prior art, and provides a hydrogel, and a detection device comprising the hydrogel which enables simple quantitative measurement of analyte in a sample fluid.

According to a first aspect, there is provided a hydrogel for use in measuring analyte levels in a fluid sample, the hydrogel comprising a pre-loaded mixture of a capture agent and a detection agent before use in measuring analyte levels in a fluid sample, wherein the mixture is randomly distributed within the hydrogel, and wherein the hydrogel has a swelling ratio of 10 to ≤50, and wherein the capture agent is covalently bonded to the hydrogel and the detection agent is physically entrapped within the hydrogel such that in use, the detection agent is able to move in and out of the hydrogel when the hydrogel swells to enable the measuring of analyte levels in the fluid sample.

The capture agent comprised in the mixture may be any suitable capture agent for the purposes of the present invention. For example, the mixture may comprise one or more different types of capture agent. The capture agent comprised in the hydrogel may depend on the type of analyte to be detected when the hydrogel is used in an analyte detection device. According to a particular aspect, the capture agent may be selected from a group consisting of: antigens, antibodies, peptides, proteins, nucleic acids, nucleic acid or peptide aptamers, ligands, receptors, and a combination thereof.

The detection agent comprised in the mixture may be any suitable detection agent for the purposes of the present invention. For example, the mixture may comprise one or more different types of detection agent. The detection agent comprised in the hydrogel may depend on the type of analyte to be detected and/or on the capture agent(s) used when the hydrogel is used in an analyte detection device. According to a particular aspect, the detection agent may be selected from the group consisting of: antigens, antibodies, peptides, proteins, nucleic acids, nucleic acid or peptide aptamers, ligands, receptors, and a combination thereof.

According to a particular aspect, the detection agent may be capable of absorbing or scattering light. According to another particular aspect, the detection agent may be labelled, for example with a light-absorbing or light-scattering material.

The hydrogel may be made of any suitable material. For example, the hydrogel may be made of a polymer. The polymer may be selected from the group consisting of, but not limited to: polyacrylamide-based, polysaccharide-based, polyvinylpyrrolidone-based polymer, copolymers, and a combination thereof. For example, the polymer may be polyacrylic acid, polyvinylalcohol, polylactic acid, polyglycolic acid, polyether, polyurethane, poly(ethylene glycol), derivatives, combinations or copolymers thereof.

The polymer from which the hydrogel is made may comprise any suitable functional group. For example, the functional group may be selected from the group consisting of, but not limited to: benzophenone, carboxyl, acid chloride, anhydride, epoxide, sulphonyl, phosphoryl, hydroxyl, phosphonate, phosphonic acid, amino acid, amides, and a combination thereof.

The hydrogel may have a suitable thickness. For example, the hydrogel may have a thickness of about 10-100 µm. In particular, the hydrogel may have a thickness of 20-80 µm, 30-70 µm, 40-60 µm, or 45-55 µm.

The hydrogel may have a suitable swelling ratio. For example, the hydrogel may have a swelling ratio of ≤50. In particular, the swelling ratio may be ≤40, ≤30, ≤25, ≤20, ≤15, ≤10, or ≤5. Even more in particular, the swelling ratio may be 10-20.

According to a second aspect, there is provided a detection device for measuring analyte levels in a sample fluid comprising the hydrogel as described in the first aspect above and an optical waveguide substrate.

The optical waveguide substrate may be any suitable substrate. According to a particular aspect, the optical waveguide substrate may be transparent.

According to a particular aspect, the optical waveguide substrate may comprise a cavity. The hydrogel may be placed within the cavity in the optical waveguide substrate. In particular, the hydrogel may be attached to a surface of the optical waveguide substrate within the cavity in the optical waveguide substrate. In use, the hydrogel may swell when contacted with a sample fluid, thereby filling up the cavity in the optical waveguide substrate.

According to a particular aspect, the detection device may further comprise a linker layer for attaching the hydrogel to a surface of the optical waveguide substrate. In particular, the hydrogel may be attached to a surface of the optical waveguide substrate within the cavity in the optical waveguide substrate by means of a linker layer.

The detection device according to any aspect of the present invention may be comprised in a chip such as a sensor chip. The sensor chip may be a disposable sensor chip.

According to a third aspect there is provided a method of measuring analyte levels in a sample fluid comprising:
- contacting the sample fluid with a hydrogel of a detection device described in the second aspect above;
- transmitting light through the optical waveguide substrate; and
- detecting a signal through the optical waveguide substrate, wherein strength of the signal detected indicates the amount of analyte in the sample fluid.

The sample fluid may be any suitable fluid. For example, the sample fluid may be a biological fluid.

According to a particular aspect, the analyte in the sample fluid may bind to the capture agent, which in turn may bind to the detection agent during the contacting. Accordingly, the signal detected during the detecting may be based on the quantity of detection agent bound to the analyte and the capture agent.

According to another particular aspect, the detection agent and the capture agent in the hydrogel may be attached to each other to form a detection agent-capture agent complex, and wherein during the contacting, the analyte may displace the detection agents in the detection agent-capture agent complex to form a complex with the capture agents. Accordingly, the signal detected during the detecting may be based on the quantity of displaced and unbound detection agents.

There is also provided a kit comprising a detection device as described above. The kit may optionally further comprise a light source and/or a detector.

### Brief Description of the Drawings

In order that the invention may be fully understood and readily put into practical effect there shall now be described by way of non-limitative example only exemplary embodiments, the description being with reference to the accompanying illustrative drawings. In the drawings:
Figure 1 shows a hydrogel according to one embodiment of the present invention;
Figure 2 shows a cross-section of a detection device according to one embodiment of the present invention;
Figure 3 shows a cross-section of a detection device according to one embodiment of the present invention;
Figure 4 shows a cross-section of a detection device according to one embodiment of the present invention;
Figure 5 shows a cross-section of a detection device according to one embodiment of the present invention; and
Figure 6 shows a cross-section of a detection device according to one embodiment of the present invention.

### Detailed description of the invention

As mentioned above, conventional assays involve multiple steps of incubation and washing which are complicated and time consuming, and may even lead to false results if the pre-treatment steps are not carried out properly. Further, such conventional assays are not suitable for integration into point-of-care (POC) systems. The present invention seeks to provide a simple and quantitative method of measuring analyte levels in a sample. In particular, the present invention seeks to reduce the number of steps involved in the conventional assay by incorporating a hydrogel system into waveguide devices, wherein all the necessary reagents required for the assay have already been pre-loaded in the hydrogel and stored in a dry state.

Accordingly, the present invention provides a hydrogel comprising a mixture of a capture agent and a detection agent, wherein the mixture is randomly distributed within the hydrogel.

For the purposes of the present invention, a hydrogel is defined as being a hydrophilic polymer network that swells when in contact with an aqueous liquid. The hydrogel may be made of any suitable polymer. According to a particular aspect, the hydrogel may be any suitable polymer which may be optically transparent and may change its swelling state according to its water content. For example, the polymer may be selected from the group consisting of, but not limited to: polyacrylamide-based, polysaccharide-based, polyvinylpyrrolidone-based polymer, copolymers, and a combination thereof. In particular, the polymer may be polyacrylic acid, polyvinylalcohol, polylactic acid, polyglycolic acid, polyether, polyurethane, poly(ethylene glycol), derivatives, combinations or copolymers thereof.

The polymer from which the hydrogel is made may comprise any suitable functional group. For example, the functional group may be selected from the group consisting of, but not limited to: benzophenone, carboxyl, acid chloride, anhydride, epoxide, sulphonyl, phosphoryl, hydroxyl, phosphonate, phosphonic acid, amino acid, amides, and a combination thereof. In particular, when the hydrogel polymer contains a functional group such as benzophenone, direct photo-cross-linking of the polymer may be enabled. This may be useful during detection of analytes in a sample fluid added to a hydrogel when the hydrogel is in use. It would be clear to a person skilled in the art that a higher concentration of benzophenone in the hydrogel may result in higher cross-linking density in the swollen states of the hydrogel when in use. Accordingly, the concentration of the benzophenone group within the hydrogel may be controlled to obtain a suitable cross-linking density of the hydrogel.

In particular, the amount of cross-linking units present in the hydrogel (i.e. ratio of cross-linking units to polymer backbone units) affects the extent of cross-linking in the hydrogel. This may lead to a hydrogel network with different cross-linking densities. The cross-linking of the hydrogel may restrict the movement of the polymer chain of the hydrogel at the hydrogel-substrate interface and within the hydrogel network. A higher cross-linking density leads to a lower swelling ratio, which in turn affects the diffusion of the analyte and/or detection agent in and out of the hydrogel network.

According to a particular aspect, the hydrogel polymer may comprise a carboxyl group. When the hydrogel polymer comprises a carboxyl group, further modification of the hydrogel polymer may be enabled. For example, the carboxyl functional group may be converted or modified in order to be able to effectively accommodate the capture agent and/or the detection agent. According to a particular aspect, the hydrogel polymer may comprise a carboxyl group which may be converted into an ester moiety. Even more in particular, the carboxyl group may be converted into N-hydroxysuccinimide (NHS) ester moiety using a mixture of a cross-linking agent such as 1-ethyl-3-[3-dimethylaminopropyl]carbodiimide (EDC) and NHS, a chemical modification reagent for converting carboxyl groups to amine-reactive NHS ester. EDC is a zero-length cross-linking agent which couples carboxyl groups to primary amines.

For the purposes of the present invention, a capture agent may be defined as an agent that can capture an analyte or analyte complex by specifically binding the analyte or a member of the complex. The member of the complex that can be bound by the capture agent may include the analyte, a carrier molecule, a carrier molecule/analyte complex, an ionic moiety, or other molecules associated with the analyte. For example, in a sandwich assay, the capture agent may be a binding partner specific for an analyte. For example, in a competitive assay, the capture agent may be a binding partner specific for a detection agent or an analyte.

The capture agent may be any suitable capture agent. For example, the mixture may comprise one or more different types of capture agent. The capture agent comprised in the hydrogel may depend on the type of analyte to be detected when the hydrogel is used in an analyte detection device. According to a particular aspect, the capture agent may be selected from the group consisting of, but not limited to: antigens, antibodies, peptides, proteins, nucleic acids, nucleic acid or peptide aptamers, ligands, receptors, and a combination thereof.

For the purposes of the present invention, a detection agent may be defined as an agent that permits the specific detection of a complex between an analyte and/or a capture agent.

The detection agent may be any suitable detection agent. For example, the mixture may comprise one or more different types of detection agent. The detection agent comprised in the hydrogel may depend on the type of analyte to be detected and/or on the capture agent(s) used when the hydrogel is used in an analyte detection device. According to a particular aspect, the detection agent may be selected from the group consisting of, but not limited to: antigens, antibodies, peptides, proteins, nucleic acids, nucleic acid or peptide aptamers, ligands, receptors, and a combination thereof.

The detection agent may be capable of providing a detectable signal. In particular, the detection agent may be capable of absorbing or scattering light, or the detection agent may be labelled with a suitable material capable of absorbing or scattering light. According to a particular aspect, the detection agent may be labelled with a light-absorbing or a light-scattering material. Any suitable label may be used for the purposes of the present invention. For example, the label may be, but not limited to, fluorophores, chromophores, upconverters, a fluorescent protein, or a combination thereof. In particular, the fluorophone may be fluorescein.

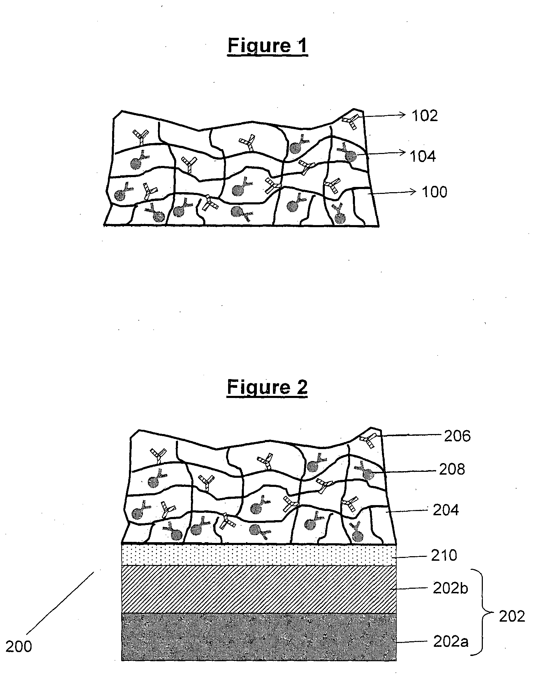

The capture agent and the detection agent may be randomly distributed within the hydrogel. For the purposes of this invention, randomly distributed is defined as a non-ordered and ungrouped manner in which the capture agent and the detection agent are scattered within the hydrogel. In particular, the capture agents and the detection agents are not separated from each other within the hydrogel. An example of a random distribution of the capture agent and the detection agent is as shown in Figure 1. Figure 1 shows a hydrogel 100 with capture agents 102 and detection agents 104 randomly distributed within the hydrogel 100.

The capture agent and the detection agent comprised in the mixture may be covalently bonded to the hydrogel or physically entrapped within the hydrogel. According to a particular aspect, the capture agent may be covalently bonded to the hydrogel polymer and the detection agent may be physically entrapped within the hydrogel. In particular, the capture agent may be covalently attached to the hydrogel polymer backbone, more particularly to the modified functional group of the hydrogel polymer. For example, the capture agents may be covalently immobilized in the hydrogel by reacting with the activated carboxyl groups such as that described above. In particular, the detection agent may be able to move inside and out of the hydrogel freely when the hydrogel swells.

The hydrogel may have properties to make it suitable for being used in a detection device. In particular, the hydrogel may have suitable cross-linking density and porosity so that in use, when a sample fluid is added to the hydrogel, the analyte in the sample fluid may rapidly diffuse in the hydrogel and the detection agents may rapidly diffuse out to enable the detection of the analyte of interest.

For example, the hydrogel may have a suitable thickness. For example, the hydrogel may.have a thickness of about 0.1-100 µm when the hydrogel is in its swollen state. In particular, the hydrogel may have a thickness of 0.5-95 µm, 1-90 µm, 5-85 µm, 10-80 µm, 20-75 µm, 30-70 µm, 40-60 µm, or 45-55 µm.

The hydrogel may have a suitable swelling ratio. Swelling ratio may be defined as the ratio of hydrogel hydrogel thickness at the swollen gel state when the hydrogel has contacted a liquid and in the dry state. The swelling ratio provides a measure of the relative change of the hydrogel thickness. For example, the hydrogel may have a swelling ratio of ≤50. In particular, the swelling ratio may be ≤40, ≤30, ≤25, ≤20, ≤15, ≤10, or ≤5. Even more in particular, the swelling ratio may be 10-20.

Polymer structures within the hydrogel such as hydrophilicity and cross-linking density may influence the swelling ratio of the hydrogel. For example, a hydrogel made from a polymer comprising more polar groups may attract more water and may therefore have a larger swelling ratio. When the cross-linking density of the hydrogel is high, the swelling ration may be low. The cross-linking density of the hydrogel may be adjusted by modifying the ratio of cross-linking units present in the hydrogel compared to the total number of polymer backbone units.

The hydrogel may be prepared by any suitable method known in the art. According to a particular embodiment, a hydrogel film comprising a dextran-based polymer is incubated in an acetate buffer (ACT, pH 4.5) containing 1-ethyl-3-(3-dimethylaminopropyl)carbodiimide (EDC, 37.5 mg/mL) and para-tetrafluorophenol sulfonate (TFPS, 10.5 mg/mL) for 20 minutes to activate the -COOH group. The activated hydrogel is then incubated with a capture agent solution such as a capture antibody solution (50 µg/mL in ACT buffer) for about one hour to enable the capture antibody to become covalently bonded to the hydrogel. After rinsing with ACT buffer, the hydrogel is incubated in ethanolamine solution (1M) to block the unreacted TFPS ester group. After about 20 minutes, the hydrogel is rinsed with ACT buffer. After a final wash with deionised water, the gel is dried in a vacuum oven for about one hour at room temperature. Subsequently, the dried hydrogel film is incubated with a detection agent solution such as a detection agent antibody solution (50 µg/mL in ACT buffer) to allow the detection antibody to diffuse into the swollen hydrogel network. Finally, the hydrogel is dried in a vacuum oven at room temperature and stored for further use, such as for use in a detection device as will be described in detail below. The hydrogel therefore comprises the covalently coupled capture antibody and the reversibly trapped detection antibody within the hydrogel.

According to another aspect of the present invention, there is provided a detection device for measuring analyte levels in a sample fluid. The detection device comprises the hydrogel as described above and an optical waveguide substrate. The detection device may be a simple tool for detection and quantification of analytes in a sample fluid. The detection device may be a portable device. In particular, the detection device may be comprised in a sensor chip. The detection device and/or the sensor chip may be a disposable device.

The detection device may be used for detecting and measuring any suitable type of analyte in any suitable sample fluid. The analyte may be an atom, molecule, group or molecules or compound of natural of synthetic origin. The analyte may be, but not limited to, antibodies, drugs, hormones, antigens, haptens, glycoproteins, glycolipids, carbohydrates, apoproteins, or cofactors. The analyte may be a biomarker. The presence or absence of a biomarker, or the increase or decrease in the concentration of a biomarker, may be associated with and/or indicative of a particular state or process.

The optical waveguide substrate may be any suitable substrate. According to a particular aspect, the optical waveguide substrate may be transparent. According to a particular aspect, the optical waveguide substrate may comprise a substrate coated with at least one layer of polymer material which has higher refractive index than the underlying layer or substrate itself. The substrate may be any suitable substrate. The layer of polymer may be optically transparent and may be able to confine the light within itself.

The layer of polymer material may be any suitable polymer material. Further, the layer of polymer material may be coated on the substrate by any suitable method well known to those skilled in the art.

According to a particular aspect, the optical waveguide substrate may comprise a cavity. In particular, the hydrogel as described above may be placed within the cavity. Even more in particular, the cavity is in the polymer layer of the optical waveguide substrate. In use, the hydrogel may swell upon contact with a sample fluid. According to a particular aspect, the hydrogel may swell to fill the cavity within which the hydrogel is placed upon contact with a sample fluid.

The hydrogel when placed within the cavity may be attached to a surface of the optical waveguide substrate. In particular, the hydrogel may be attached to the surface of the polymer layer of the optical waveguide substrate. The hydrogel may be placed within the cavity by any suitable method. For example, the hydrogel may be placed within the cavity by, but not limited to, doctor blading, dip coating, spray coating or spin coating. The spin coating may be with or without the aid of a shadow mask.

According to a particular aspect, the hydrogel may be placed on the surface of the optical waveguide substrate which may not comprise a cavity. In particular, when the optical waveguide substrate comprises a substrate and a polymer layer coated on the substrate, the hydrogel may be placed on the surface of the polymer layer.

When the adhesion between the hydrogel and the surface of the optical waveguide substrate is weak or insufficient, the detection device may further comprise a linker layer for attaching the hydrogel to the surface of the optical waveguide substrate. In particular, the hydrogel may be attached to a surface of the optical waveguide substrate within the cavity in the optical waveguide substrate by means of a linker layer.

The linker layer may be of any suitable material. For example, the linker layer may be formed from a material depending on the surface of the optical waveguide substrate on which the linker layer is formed. According to a particular aspect, the linker layer may be of the same base polymer as the polymer layer of the optical waveguide substrate but with a higher density of cross-linking units.

The linker layer may be covalently or non-covalently attached to the surface of the optical waveguide substrate. Non-covalent attachment may be by any suitable secondary interaction, including but not limited to hydrophobic interactions, hydrogen bonding, van der Waals forces, ionic bonds, and the like. According to a particular aspect, the linker layer may be cross-linked to the polymer layer of the optical waveguide substrate through covalent bonding.

The linker layer may be deposited on the surface of the optical waveguide substrate by any suitable method. For example, the linker layer may be deposited as a self-assembled monolayer, by reactive plasma etching, corona discharge treatment, plasma deposition process, spin coating, dip coating, spray coating, or the linker layer may be created by modification of the surface of the optical waveguide substrate by chemical reaction.

Figure 2 shows a cross-section of one embodiment of the detection device of the present invention. In particular, there is provided a detection device 200 comprising an optical waveguide substrate 202 and a hydrogel 204 comprising capture agents 206 and detection agents 208. The capture agents 206 and the detection agents 208 are randomly distributed within the hydrogel 204. The optical waveguide substrate 202 comprises a substrate 202a and a polymer layer 202b. The detection device 200 also comprises a linker layer 210 between the polymer layer 202b and the hydrogel 204 to enable adhesion of the hydrogel 204 to the optical waveguide substrate 202.

The detection device according to any aspect of the present invention may be comprised in a chip, such as a sensor chip or the like. The sensor chip may be a disposable sensor chip.

The detection device may be used in any form of heterogeneous assay. For example, the detection device may be used in a sandwich-type assay, in a displacement assay or in a competitive assay during the detection of analytes in a fluid sample.

The detection device may be made by any suitable method known in the art. In particular, the hydrogel may be incorporated into the detection device by any suitable method. For example, selective deposition of the hydrogel by cross-linking through a stencil mask may be used.

According to a particular embodiment, a waveguide chip may be attached to a glass plate holder and a stencil mask may be adhered to the optical waveguide substrate. Poly(*N*-isopropylacrylamide) (PNIPAAM) (0.5% w/v in ethanol) may be spin coated at about 2500 rpm and dried at 50°C in a vacuum oven for about 3 hours. The polymer film may then be cross-linked under 254 nm UV light at a dose of 15 J/cm². A dextran hydrogel layer may be formed by spin coating about 15-20% w/v dextran polymer solution at about 2500 rpm over the cross-linked PNIPAAM polymer film. The dextran hydrogel layer may be dried at 50°C in a vacuum oven for about 3 hours and cross-linked under 254 nm UV light at a dose of 8 J/cm². The thickness of the hydrogel formed may be about 1-2 µm in the dry state and about 10-20 µm in the swollen state. The coated optical waveguide substrate may then be washed with deionised water and ethanol to remove any non cross-linked polymer. The dextran hydrogel may only adhere to the hydrophilic area defined by the PNIPAAM polymer film.

The present invention also provides a kit comprising the detection device as described above. The kit may further comprise a light source and/or a detector. The light source may be any suitable light source, such as but not limited to a UV light source, incandescent light source, LED light source, or laser light source. The detector may be any suitable detector. For example, the detector may be, but not limited to, a fluorescence detector, photodiode, or a photon multiplier tube (PMT) detector.

According to another aspect of the present invention, there is provided a method of measuring analyte levels in a sample fluid comprising:
- contacting the sample fluid with a hydrogel of a detection device according to any aspect of the present invention described above;
- transmitting light through the optical waveguide substrate; and
- detecting a signal through the optical waveguide substrate, wherein strength of the signal detected indicates the amount of analyte in the sample fluid.

The sample fluid may be any suitable sample fluid. According to a particular aspect, the sample fluid may be a biological fluid. For example, the sample fluid may be, but not limited to, a sample of blood, serum, plasma, interstitial fluid, cerebral spinal fluid, lymph fluid, ocular fluid, saliva, oral fluid, urine, sweat, excretions, exudates, and the like. The sample fluid may be collected or obtained from an animal, human or plant using methods well known to those skilled in the art.

The analyte in the sample fluid may be any suitable analyte. For example, the analyte may be an atom, molecule, group or molecules or compound of natural of synthetic origin. The analyte may be, but not limited to, antibodies, drugs, hormones, antigens, haptens, glycoproteins, glycolipids, carbohydrates, apoproteins, or cofactors be associated with and/or indicative of a particular state or process.

According to a particular aspect, the analyte in the sample fluid may bind to the capture agent during the contacting. The analyte may form an analyte-capture agent complex which may then bind to the detection agent, thereby forming a detection agent-analyte-capture agent complex. Accordingly, during the detecting, the signal detected may be based on the quantity of the detection agent bound to the analyte and capture agent.

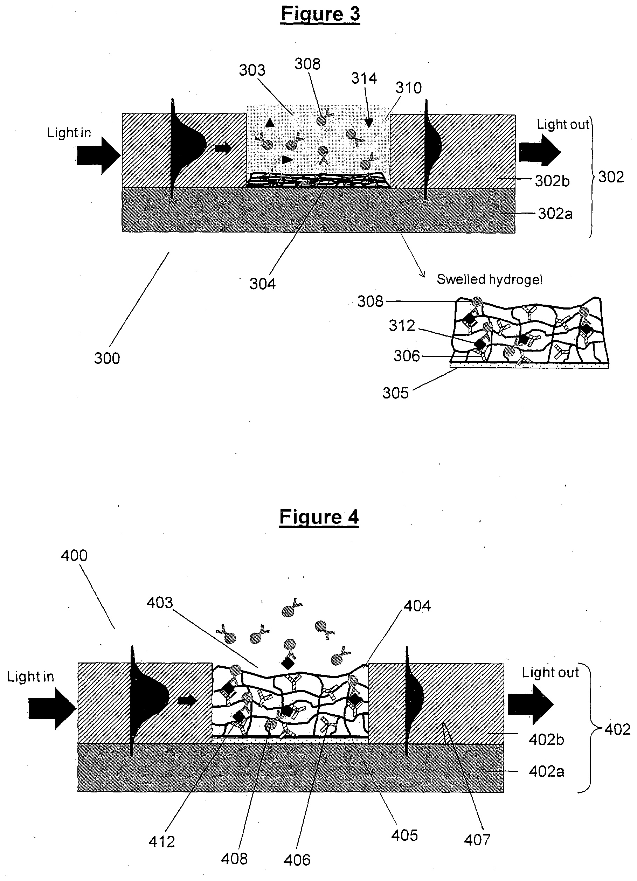

One embodiment of the detection method described above is shown in Figure 3. Figure 3 provides a detection device 300 comprising an optical waveguide substrate 302 which comprises a substrate 302a and a polymer layer 302b on the surface of the substrate 302a as the optical waveguide. There is also provided a hydrogel 304 in a cavity 303 in the polymer layer 302b. The hydrogel is attached to the surface of the substrate 302a by means of a linker layer 305. The hydrogel 304 comprises capture agents 306 and detection agents 308 randomly distributed within the hydrogel 304. In particular, the capture agents 306 are covalently bonded to the hydrogel 304, while the detection agents 308 are physically dispersed within the hydrogel 304.

In use, a sample fluid 310 is added into the cavity 303 in which the hydrogel 304 is placed. The sample fluid 310 comprises target analytes 312 and impurities 314. When the hydrogel 304 contacts the sample fluid 310, the hydrogel 304 swells and the target analytes 312 in the sample fluid diffuse into the hydrogel 304 and bind to the capture agents 306 to form an analyte-capture agent complex. In the presence of the target analyte 312, the detection agents 308 are retained in the hydrogel 304 and bind to the analyte-capture agent complex to form a detection agent-analyte-capture agent complex. The unbound detection agents 308 are released to the sample fluid 310 out of the hydrogel 304. When light (as shown by the black arrows) is transmitted through the polymer layer 302b of the optical waveguide substrate 302, a detector (not shown) placed on the side of the device 300 opposite the side from which the light is transmitted detects a signal from the unbound detection agents 308 released from the hydrogel 304. In particular, the detected signal is proportional to the amount of the detection agents 308 released into the sample fluid 310, which is inversely proportional to the amount of target analyte 312 in the sample fluid 310 added into the cavity 303. In this way, a quantitative measure of the target analyte 312 in the sample fluid 310 may be carried out using the hydrogel and detection device of the present invention.

Another embodiment of the detection method described above is shown in Figure 4. Figure 4 provides a detection device 400 comprising an optical waveguide substrate 402 which comprises a substrate 402a and a polymer layer 402b on the surface of the substrate 402a as the optical waveguide. There is also provided a hydrogel 404 in a cavity 403 in the polymer layer 402b. The hydrogel 404 is attached to a surface 407 of the polymer layer 402b by means of a linker layer 405. The hydrogel 404 comprises capture agents 406 and detection agents 408 randomly distributed within the hydrogel 404. In particular, the capture agents 406 are covalently bonded to the hydrogel 404, while the detection agents 408 are physically dispersed within the hydrogel 404.

In use, a sample fluid is added into the cavity 403 in which the hydrogel 404 is placed. The sample fluid comprises target analytes 412. When the hydrogel 404 contacts the sample fluid, the hydrogel 404 swells and occupies the entire space within the cavity 403. The target analytes 412 in the sample fluid diffuse into the hydrogel 404 and bind to the capture agents 406 to form an analyte-capture agent complex. In the presence of the target analyte 412, the detection agents 408 are retained in the hydrogel 404 and bind to the analyte-capture agent complex to form a detection agent-analyte-capture agent complex. The unbound detection agents 408 are released out of the hydrogel 404 and washed away during an optional washing step of the detection device 400. When light (as shown by the black arrows) is transmitted through the polymer layer 402b of the optical waveguide substrate 402, a detector (not shown) placed on one side of the device 400 opposite the side from which the light is transmitted detects a signal from the detection agents 408 which are bound to the analyte-capture agent complex. In particular, the detected signal is proportional to the amount of the detection agents 408 retained within the hydrogel 404 and which are bound to the analyte-capture agent complex. In this way, a quantitative measure of the target analyte 412 in the sample fluid added to the hydrogel 404 may be carried out using the hydrogel and detection device of the present invention.

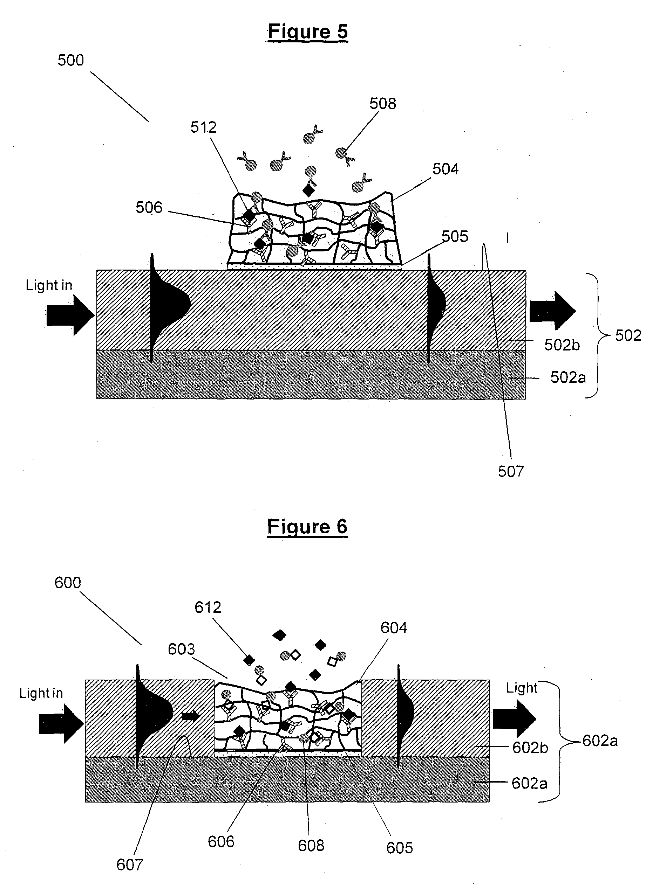

Another embodiment of the detection method described above is shown in Figure 5. Figure 5 provides a detection device 500 comprising an optical waveguide substrate 502 which comprises a substrate 502a and a polymer layer 502b on the surface of the substrate 502a as the optical waveguide. There is also provided a hydrogel 504 on the polymer layer 502b. The hydrogel 504 is attached to a surface 507 of the polymer layer 502b by means of a linker layer 505. The hydrogel 504 comprises capture agents 506 and detection agents 508 randomly distributed within the hydrogel 504. In particular, the capture agents 506 are covalently bonded to the hydrogel 504, while the detection agents 508 are physically dispersed within the hydrogel 504.

In use, a sample fluid is added to the hydrogel 504. The sample fluid comprises target analytes 512. When the hydrogel 504 contacts the sample fluid, the hydrogel 504 swells. The target analytes 512 in the sample fluid diffuse into the hydrogel 504 and bind to the capture agents 506 to form an analyte-capture agent complex. In the presence of the target analyte 512, the detection agents 508 are retained in the hydrogel 504 and bind to the analyte-capture agent complex to form a detection agent-analyte-capture agent complex. The unbound detection agents 508 are released out of the hydrogel 504 and washed away during an optional washing step of the detection device 500 after the contacting of the sample fluid with the hydrogel 504. When light (as shown by the black arrows) is transmitted through the polymer layer 502b of the optical waveguide substrate 502, a detector (not shown) placed on one side of the device 500 opposite the side from which the light is transmitted detects a signal formed by the interaction of the detection agents 508 which are bound to the analyte-capture agent complex with the evanescent wave generated at the surface of the polymer layer 502b of the optical waveguide substrate 502. In particular, the detected signal is proportional to the amount of the detection agents 508 retained within the hydrogel 504 and which are bound to the analyte-capture agent complex. In this way, a quantitative measure of the target analyte 512 in the sample fluid added to the hydrogel 504 may be carried out using the hydrogel and detection device of the present invention.

According to another particular aspect, the detection agent and the capture agent may be attached to each other to form a detection agent-capture agent complex within the hydrogel. Accordingly, when the sample fluid contacts the hydrogel during the contacting, the analyte in the sample fluid may diffuse into the hydrogel and may displace the detection agent from the detection agent-capture agent complex to form an analyte-capture agent complex. Therefore, during the detecting, the signal detected may be based on the quantity of the displaced detection agent.

One embodiment of the detection method described above is as shown in Figure 6. In particular, there is provided a detection device 600 comprising an optical waveguide substrate 602 which comprises a substrate 602a and a polymer layer 602b on the surface of the substrate 602a as the optical waveguide. There is also provided a hydrogel 604 in a cavity 603 in the polymer layer 602b. The hydrogel 604 is attached to a surface 607 of the polymer layer 602b by means of a linker layer 605. The hydrogel 604 comprises capture agents 606 and labelled detection agents 608 bound to each other through affinity binding to form detection agent-capture agent complexes. These detection agent-capture agent complexes are randomly distributed within the hydrogel 604.

In use, a sample fluid is added into the cavity 603 in which the hydrogel 604 is placed. The sample fluid comprises target analytes 612. When the hydrogel 604 contacts the sample fluid, the hydrogel 604 swells and the target analytes 612 in the sample fluid diffuse into the hydrogel 604 and displace the labelled detection agents 608 from the detection agent-capture agent complex to form an analyte-capture agent complex. The displaced labelled detection agents 608 are released to the sample fluid and out of the hydrogel 604. When light (as shown by the black arrows) is transmitted through the polymer layer 602b of the optical waveguide substrate 602, a detector (not shown) placed on the side of the device 600 opposite the side from which the light is transmitted detects a signal from the displaced and unbound labelled detection agents 608 released from the hydrogel 604. In particular, the detected signal is proportional to the amount of the labelled detection agents 608 released into the sample fluid, which is proportional to the amount of target analyte 612 in the sample fluid added into the cavity 603. In this way, a quantitative measure of the target analyte 612 in the sample fluid may be carried out using the hydrogel and detection device 600 of the present invention.

The detection device of the present invention provides a simple optical method of quantitatively measuring the amount of target analyte in a sample fluid without requiring any pre-treatment of the sample fluid. Further, the amount of sample fluid required is also minimal in order to perform the method of measuring the amount of target analyte. In particular, the hydrogel comprised in the detection device provides a way of pre-storing all the necessary reagents required to perform the method. In this way, the shelf life of the detection device may be extended. The present invention therefore provides a suitable medium for storing dry reagents with increased stability in the form of the hydrogel, which in turn allows rapid uptake of a sample fluid by osmotic pressure to allow the quantitative determination of target analytes in the sample fluid. By relying on osmotic pressure for the target analyte to enter the hydrogel in the detection device, the need for washing or liquid transfer steps is eliminated, thereby eliminating the need for expensive or complex microfluidics, which reduces the cost for analysing a sample fluid.

Whilst the foregoing description has described exemplary embodiments, it will be understood by those skilled in the technology concerned that many variations in details of design, construction and/or operation may be made without departing from the present invention.

## Claims

1. A hydrogel (100;204;304;404;504;604) for use in measuring analyte levels in a fluid sample (310), the hydrogel comprising a pre-loaded mixture of a capture agent (102;206;306;406;506;606) and a detection agent (104;208;308;408;508;608) before use in measuring analyte (312;412;512;612) levels in a fluid sample, wherein the mixture is randomly distributed within the hydrogel, and wherein the hydrogel has a swelling ratio of 10 to ≤50, and **characterised in that** the capture agent is covalently bonded to the hydrogel and the detection agent is physically entrapped within the hydrogel such that in use, the detection agent is able to move in and out of the hydrogel when the hydrogel swells to enable the measuring of analyte levels in the fluid sample.

2. The hydrogel according to any preceding claim, wherein: the capture agent is selected from a group consisting of: antigens, antibodies, peptides, proteins, nucleic acids, nucleic acid or peptide aptamers, ligands, receptors, and a combination thereof.

3. The hydrogel according to any preceding claim, wherein: the detection agent is selected from a group consisting of: antigens, antibodies, peptides, proteins, nucleic acids, nucleic acid or peptide aptamers, ligands, receptors, and a combination thereof.

4. The hydrogel according to any preceding claim, wherein the detection agent is labelled with a light-absorbing or light-scattering material.

5. A detection device (200;300;400;500;600) for measuring analyte (312;412;512;612) levels in a sample fluid (310), the detection device comprising: an optical waveguide substrate (202;302;402;502;602); and a hydrogel (100;204;304;404;504;604) according to any of the preceding claims.

6. The detection device according to claim 5, wherein the optical waveguide substrate is transparent.

7. The detection device according to claim 5 or 6, wherein the optical waveguide substrate comprises a cavity within which the hydrogel is placed.

8. The detection device according to claim 7, further comprising a linker layer for attaching the hydrogel to a surface of the optical waveguide substrate within the cavity.

9. The detection device according to claim 7 or 8, wherein the hydrogel swells when contacted with a sample fluid thereby filling up the cavity.

10. The detection device according to any of claims 5 to 9, wherein the detection device is comprised in a sensor chip.

11. A method of measuring analyte (312;412;512;612) levels in a sample fluid (310) comprising:
- contacting the sample fluid with a hydrogel (100;204;304;404;504;604) of a detection device (200;300;400;500;600) according to any of claims 5 to 10;
- transmitting light through the optical waveguide substrate (202;302;402;502;602); and
- detecting a signal through the optical waveguide substrate, wherein strength of the signal detected indicates the amount of analyte in the sample fluid.

12. The method according to claim 11, wherein during the contacting, the analyte binds to the capture agent which in turn binds to the detection agent.

13. The method according to claim 12, wherein the signal detected is based on the quantity of detection agents bound to the analyte and capture agents.

14. The method according to claim 11, wherein the detection agent and the capture agent in the hydrogel are attached to each other to form a detection agent-capture agent complex, and wherein during the contacting, the analyte displaces the detection agents in the detection agent-capture agent complex to form a complex with the capture agents.

15. The method according to claim 14, wherein the signal detected is based on the quantity of the displaced detection agents.

## Patentansprüche

1. Hydrogel (100;204;304;404;504;604) zur Verwendung beim Messen der Gehalte von Analyt in einer Fluidprobe (310), wobei das Hydrogel eine vorbeladene Mischung eines Einfangmittels (102;206;306;406;506;606) und eines Detektionsmittels (104;208;308;408;508;608) umfasst, bevor es beim Messen der Gehalte von Analyt (312;412;512;612) in einer Fluidprobe verwendet wird, wobei die Mischung regellos in dem Hydrogel verteilt ist, und wobei das Hydrogel ein Quellverhältnis von 10 bis ≤50 aufweist, und **dadurch gekennzeichnet, dass** das Einfangmittel kovalent an dem Hydrogel gebunden ist und das Detektionsmittel physikalisch in dem Hydrogel derart eingeschlossen ist, dass das Detektionsmittel bei Verwendung in der Lage ist, in das Hydrogel ein- und auszutreten, wenn das Hydrogel quillt, um das Messen von Gehalten von Analyt in der Fluidprobe zu ermöglichen.

2. Hydrogel nach einem der vorgehenden Ansprüche, wobei: das Einfangmittel ausgewählt ist aus der Gruppe bestehend aus: Antigenen, Antikörpern, Peptiden, Proteinen, Nucleinsäuren, Nucleinsäure- oder Peptid-Aptameren, Liganden, Rezeptoren und einer Kombination davon.

3. Hydrogel nach einem der vorgehenden Ansprüche, wobei: das Detektionsmittel ausgewählt ist aus der Gruppe bestehend aus: Antigenen, Antikörpern, Peptiden, Proteinen, Nucleinsäuren, Nucleinsäure- oder Peptid-Aptameren, Liganden, Rezeptoren und einer Kombination davon.

4. Hydrogel nach einem der vorgehenden Ansprüche, wobei das Detektionsmittel markiert ist mit einem Licht absorbierenden oder Licht streuenden Material.

5. Detektionsvorrichtung (200;300;400;500;600) zum Messen der Gehalte von Analyt (312;412;512;612) in einem Probenfluid (310), wobei die Detektionsvorrichtung umfasst: ein Lichtwellenleitersubstrat (202;302;402;502;602); und ein Hydrogel (100;204;304;404;504;604) nach einem der vorgehenden Ansprüche.

6. Detektionsvorrichtung nach Anspruch 5, wobei das Lichtwellenleitersubstrat transparent ist.

7. Detektionsvorrichtung nach Anspruch 5 oder 6, wobei das Lichtwellenleitersubstrat einen Hohlraum umfasst, in dessen Innerem das Hydrogel angeordnet ist.

8. Detektionsvorrichtung nach Anspruch 7, ferner umfassend eine Linkerschicht zum Anbringen des Hydrogels an einer Oberfläche des Lichtwellenleitersubstrats im Inneren des Hohlraums.

9. Detektionsvorrichtung nach Anspruch 7 oder 8, wobei das Hydrogel quillt, wenn es mit einem Probenfluid in Kontakt gebracht wird, wodurch es den Hohlraum ausfüllt.

10. Detektionsvorrichtung nach einem der Ansprüche 5 bis 9, wobei die Detektionsvorrichtung in einem Sensorchip enthalten ist.

11. Verfahren zum Messen von Gehalten von Analyt (312;412;512;612) in einem Probenfluid (310) umfassend:
- Kontaktieren des Probenfluids mit einem Hydrogel (100;204;304;404;504;604) einer Detektionsvorrichtung (200;300;400;500;600) nach einem der Ansprüche 5 bis 10;
- Durchleiten von Licht durch das Lichtwellenleitersubstrat (202;302;402;502;602); und
- Detektieren eines Signals durch das Lichtwellenleitersubstrat, wobei die Stärke des detektierten Signals die Menge von Analyt in dem Probenfluid anzeigt.

12. Verfahren nach Anspruch 11, wobei während des Kontaktierens der Analyt an dem Einfangmittel bindet, das wiederum an dem Detektionsmittel bindet.

13. Verfahren nach Anspruch 12, wobei das detektierte Signal auf der Menge an Detektionsmittel beruht, das an dem Analyten und den Einfangmitteln gebunden ist.

14. Verfahren nach Anspruch 11, wobei das Detektionsmittel und das Einfangmittel in dem Hydrogel aneinander angelagert sind, um einen Detektionsmittel-Einfangmittel-Komplex zu bilden, und wobei während des Kontaktierens der Analyt die Detektionsmittel in dem Detektionsmittel-Einfangmittel-Komplex verdrängt, um einen Komplex mit den Einfangmitteln zu bilden.

15. Verfahren nach Anspruch 14, wobei das detektierte Signal auf der Menge der verdrängten Detektionsmittel beruht.

## Revendications

1. Hydrogel (100; 204; 304; 404; 504; 604) pour une utilisation dans la mesure de niveaux d'analyte dans un échantillon de fluide (310), l'hydrogel comprenant un mélange pré-chargé d'un agent de capture (102; 206; 306; 406; 506; 606) et d'un agent de détection (104; 208; 308; 408; 508; 608) avant une utilisation dans la mesure de niveaux d'analyte (312; 412; 512; 612) dans un échantillon de fluide, où le mélange est distribué aléatoirement dans l'hydrogel et où l'hydrogel a un rapport de gonflement de 10 à ≤50 et **caractérisé en ce que** l'agent de capture est lié par covalence à l'hydrogel et l'agent de détection est piégé physiquement dans l'hydrogel de sorte que, pendant l'utilisation, l'agent de détection est capable d'entrer et de sortir de l'hydrogel lorsque l'hydrogel gonfle pour permettre la mesure des niveaux d'analyte dans l'échantillon de fluide.

2. Hydrogel selon l'une quelconque des revendications précédentes, dans lequel:
l'agent de capture est choisi dans le groupe constitué de: antigènes, anticorps, peptides, protéines, acides nucléiques, aptamères d'acides nucléiques ou de peptides, ligands, récepteurs et une combinaison de ceux-ci.

3. Hydrogel selon l'une quelconque des revendications précédentes, dans lequel:
l'agent de détection est choisi dans le groupe constitué de: antigènes, anticorps, peptides, protéines, acides nucléiques, aptamères d'acides nucléiques ou de peptides, ligands, récepteurs et une combinaison de ceux-ci.

4. Hydrogel selon l'une quelconque des revendications précédentes, dans lequel l'agent de détection est marqué avec un matériau absorbant la lumière ou diffusant la lumière.

5. Dispositif de détection (200; 300; 400; 500; 600) pour la mesure de niveaux d'analyte (312; 412; 512; 612) dans un fluide échantillon (310), le dispositif de détection comprenant: un substrat de guide d'onde optique (202; 302; 402; 502; 602); et un hydrogel (100; 204; 304; 404; 504; 604) selon l'une quelconque des revendications précédentes.

6. Dispositif de détection selon la revendication 5, dans lequel le substrat de guide d'onde optique est transparent.

7. Dispositif de détection selon la revendication 5 ou 6, dans lequel le substrat de guide d'onde optique comprend une cavité dans laquelle l'hydrogel est placé.

8. Dispositif de détection selon la revendication 7, comprenant en outre une couche liante pour fixer l'hydrogel à une surface du substrat de guide d'onde optique dans la cavité.

9. Dispositif de détection selon la revendication 7 ou 8, dans lequel l'hydrogel gonfle lorsqu'il est en contact avec un fluide échantillon remplissant ainsi la cavité.

10. Dispositif de détection selon l'une quelconque des revendications 5 à 9, où le dispositif de détection est compris dans une puce de capteur.

11. Méthode pour la mesure de niveaux d'analyte (312; 412; 512; 612) dans un fluide échantillon (310) comprenant:
- la mise en contact du fluide échantillon avec un hydrogel (100; 204; 304; 404; 504; 604) d'un dispositif de détection (200; 300; 400; 500; 600) selon l'une quelconque des revendications 5 à 10;
- la transmission d'une lumière à travers le substrat de guide d'onde optique (202; 302; 402; 502; 602); et
- la détection d'un signal par le substrat de guide d'onde optique, où l'intensité du signal détecté indique la quantité d'analyte dans le fluide échantillon.

12. Méthode selon la revendication 11, dans laquelle durant la mise en contact, l'analyte se lie à l'agent de capture qui à son tour se lie à l'agent de détection.

13. Méthode selon la revendication 12, dans laquelle le signal détecté est basé sur la quantité d'agents de détection liés à l'analyte et aux agents de capture.

14. Méthode selon la revendication 11, dans laquelle l'agent de détection et l'agent de capture dans l'hydrogel sont fixés l'un à l'autre pour former un complexe d'agent de détection-agent de capture et dans laquelle durant la mise en contact, l'analyte déplace les agents de détection dans le complexe d'agent de détection-agent de capture pour former un complexe avec les agents de capture.

15. Méthode selon la revendication 14, dans laquelle le signal détecté est basé sur la quantité des agents de détection déplacés.
